# EUROPEAN PATENT APPLICATION

(11) **EP 4 082 419 A1**
(43) Date of publication of application: **02.11.2022**
(21) Application number: 20907448.3
(22) Date of filing: 25.11.2020
(51) Int. Cl.: A61B 1/00, A61B 1/045, A61B 1/06, H04N 5/225, H04N 5/232, H04N 7/18

(54) **ENDOSCOPE SYSTEM AND METHOD FOR OPERATING SAME**

(30) Priority: 26.12.2019 JP 2019236714
(71) Applicant: FUJIFILM Corporation, Tokyo 106-8620 (JP)
(72) Inventor: KUBO, Masahiro, Ashigarakami-gun, Kanagawa 258-8538 (JP)
(74) Representative: Hughes, Andrea Michelle
(86) International application number: PCT/JP2020/043833
(87) International publication number: WO 2021/131468

(57) **Abstract**

There are provided an endoscope system and a method of operating the endoscope system that can switch a mode to a multi-light emission mode without imposing a burden on a user in a case where a mode is switched to a mono-light emission mode where only specific illumination light is emitted from a multi-light emission mode where an object to be observed is illuminated with a plurality of types of illumination light while the plurality of types of illumination light are automatically switched.

In a case where a predetermined mono-light emission switching condition is satisfied and a mode is thus automatically switched to the mono-light emission mode from the multi-light emission mode, a mode is automatically switched to the multi-light emission mode from the mono-light emission mode in a case where a preset multi-light emission restart condition is satisfied.

## Description

### BACKGROUND OF THE INVENTION

### 1. Field of the Invention

The present invention relates to an endoscope system and a method of operating the endoscope system that illuminate a subject with a plurality of types of illumination light having different wavelength ranges while switching the plurality of types of illumination light and display observation images corresponding to the respective types of illumination light while switching the observation images.

### 2. Description of the Related Art

In recent years, an endoscope system comprising a light source device, an endoscope, and a processor device has been widely used in a medical field. In the endoscope system, an object to be observed is irradiated with illumination light from an endoscope, and the image of the object to be observed is displayed on a display on the basis of RGB image signals that are obtained in a case where the image of the object to be observed, which is being illuminated with the illumination light, is picked up by an image pickup element of the endoscope.

Further, in recent years, an object to be observed has been illuminated with a plurality of types of illumination light having different wavelength ranges to obtain much diagnosis information from the object to be observed. For example, in WO2019/093356A, first illumination light and second illumination light are emitted while being switched according to a specific light emission pattern, and a first observation image and a second observation image are displayed on a display while being switched according to a specific display pattern.

### SUMMARY OF THE INVENTION

In WO2019/093356A, a multi-light emission mode where an object to be observed is illuminated with a plurality of types of illumination light while the plurality of types of illumination light are switched may not be suitable for a case where it is difficult for illumination light to reach an object to be observed since a distal end part of an endoscope is moved unintentionally or the object to be observed is out of focus, and the like. Accordingly, in a case where a preset designated condition is satisfied, a mode is adapted to be switched to a mono-light emission mode where only specific illumination light is emitted from the multi-light emission mode.

However, in a case where an image pickup condition about the object to be observed is temporarily changed and immediately restored to an image pickup condition suitable for the multi-light emission mode even though a mode is switched to the mono-light emission mode, a user often wants to observe the object to be observed in the multi-light emission mode again. In such cases, it is very cumbersome and stressful for the user to manually reselect the multi-light emission mode.

An object of the present invention is to provide an endoscope system and a method of operating the endoscope system that can switch a mode to a multi-light emission mode without imposing a burden on a user in a case where a mode is switched to a mono-light emission mode where only specific illumination light is emitted from a multi-light emission mode where an object to be observed is illuminated with a plurality of types of illumination light while the plurality of types of illumination light are automatically switched.

According to an aspect of the present invention, there is provided an endoscope system comprising: a plurality of semiconductor light sources that emit light having wavelength ranges different from each other; a light source processor that controls the plurality of semiconductor light sources and performs a control related to a mono-light emission mode where only specific illumination light having a specific light emission ratio is emitted and a control related to a multi-light emission mode where a plurality of types of illumination light, which include first illumination light having a first light emission ratio and second illumination light having a second light emission ratio different from the first light emission ratio, are emitted while being switched according to a specific light emission pattern; and an image control processor. In a case where a predetermined mono-light emission switching condition is satisfied and a mode is thus automatically switched to the mono-light emission mode from the multi-light emission mode, the image control processor automatically switches a mode to the multi-light emission mode from the mono-light emission mode in a case where a preset multi-light emission restart condition is satisfied.

It is preferable that the image control processor automatically switches a mode to the multi-light emission mode from the mono-light emission mode in a case where the multi-light emission restart condition is satisfied and a restart allowable condition is satisfied, and prohibits a mode from being automatically switched to the multi-light emission mode in a case where the multi-light emission restart condition is satisfied and the restart allowable condition is not satisfied.

It is preferable that the restart allowable condition is a case where a user does not select the mono-light emission mode. It is preferable that the restart allowable condition is a case where a restart allowable time does not elapse yet after a mode is switched to the mono-light emission mode. It is preferable that the restart allowable condition is a case where the multi-light emission mode is implementable. It is preferable that the endoscope system further comprises a magnification change unit that is used to change a magnification of an object to be observed, and the restart allowable condition is a case where use or non-use of the magnification change unit is not switched. It is preferable that the restart allowable condition is capable of being set by a user.

It is preferable that the multi-light emission restart condition is that a change in an image pickup condition about an object to be observed is within an allowable range. It is preferable that the multi-light emission restart condition is a condition of a combination of at least one of a case where the object to be observed returns to a first portion within a certain period of time after being changed to a second portion from the first portion or a case where the object to be observed returns to the second portion within a certain period of time after being changed to the first portion from the second portion, a case where brightness of the object to be observed is equal to or higher than a first brightness threshold value or is equal to or lower than a second brightness threshold value larger than the first brightness threshold value, a case where a variation of magnification of the object to be observed is less than a magnification threshold value in a case where the magnification of the object to be observed is changed, a case where a variation of an observation distance is equal to or smaller than a distance threshold value, or a case where a shake amount of an observation image obtained from image pickup of the object to be observed is equal to or smaller than a shake-amount threshold value.

It is preferable that the image control processor performs a control to display a specific observation image, which is obtained from image pickup of an object to be observed illuminated with the specific illumination light, on a display in a case of the mono-light emission mode and performs a control to display a plurality of observation images including a first observation image, which is obtained from image pickup of the object to be observed illuminated with the first illumination light, and a second observation image, which is obtained from image pickup of the object to be observed illuminated with the second illumination light, on the display while switching the plurality of observation images according to a specific display pattern in a case of the multi-light emission mode.

According to another aspect of the present invention, there is provided a method of operating an endoscope system. The endoscope system includes: a plurality of semiconductor light sources that emit light having wavelength ranges different from each other; a light source processor that controls the plurality of semiconductor light sources and performs a control related to a mono-light emission mode where only specific illumination light having a specific light emission ratio is emitted and a control related to a multi-light emission mode where a plurality of types of illumination light, which include first illumination light having a first light emission ratio and second illumination light having a second light emission ratio different from the first light emission ratio, are emitted while being switched according to a specific light emission pattern; and an image control processor. In a case where a predetermined mono-light emission switching condition is satisfied and a mode is thus automatically switched to the mono-light emission mode from the multi-light emission mode, the image control processor automatically switches a mode to the multi-light emission mode from the mono-light emission mode in a case where a preset multi-light emission restart condition is satisfied.

According to the present invention, it is possible to switch a mode to a multi-light emission mode without imposing a burden on a user in a case where a mode is switched to a mono-light emission mode where only specific illumination light is emitted from a multi-light emission mode where an object to be observed is illuminated with a plurality of types of illumination light while the plurality of types of illumination light are automatically switched.

### BRIEF DESCRIPTION OF THE DRAWINGS

Fig. 1 is a diagram showing the appearance of an endoscope system according to a first embodiment.
Fig. 2 is a block diagram showing the functions of the endoscope system according to the first embodiment.
Fig. 3 is a graph showing the emission spectra of violet light V, blue light B, green light G, and red light R.
Fig. 4 is an image diagram showing a normal image.
Fig. 5 is a graph showing the emission spectrum of first illumination light that includes violet light V, blue light B, green light G, and red light R.
Fig. 6 is an image diagram showing a first observation image.
Fig. 7 is a graph showing the emission spectrum of second illumination light that includes violet light V, blue light B, green light G, and red light R.
Fig. 8 is an image diagram showing a second observation image.
Fig. 9 is a diagram illustrating the light emission of the first illumination light and the second illumination light and the display of the first and second observation images in a multi-light emission mode.
Fig. 10 is a diagram illustrating that a mode is automatically switched to a mono-light emission mode from the multi-light emission mode.
Fig. 11 is a diagram illustrating that a mode is automatically switched to the mono-light emission mode from the multi-light emission mode in a case where the use time of the multi-light emission mode is equal to or longer than a time threshold value.
Fig. 12 is a diagram illustrating that a mode is automatically switched to the mono-light emission mode from the multi-light emission mode in a case where the number of times of storage of a static image is equal to or larger than a number-of-times threshold value.
Fig. 13 is a block diagram showing the functions of an image-pickup-condition acquisition unit.
Fig. 14 is a diagram illustrating that a mode is automatically switched to the mono-light emission mode from the multi-light emission mode in a case where an observation portion is changed to a second portion from a first portion or in a case where an observation portion is changed to the first portion from the second portion.
Fig. 15 is a diagram illustrating that a mode is automatically switched to the mono-light emission mode from the multi-light emission mode in a case where brightness is equal to or lower than a first brightness threshold value or brightness is equal to or higher than a second brightness threshold value.
Fig. 16 is a diagram illustrating that a mode is automatically switched to the mono-light emission mode from the multi-light emission mode in a case where the variation of magnification exceeds a magnification threshold value.
Fig. 17 is a diagram illustrating that a mode is automatically switched to the mono-light emission mode from the multi-light emission mode in a case where the variation of an observation distance exceeds a distance threshold value.
Fig. 18 is a diagram illustrating that a mode is automatically switched to the mono-light emission mode from the multi-light emission mode in a case where a shake amount exceeds a shake-amount threshold value.
Fig. 19 is an image diagram showing a mono-light emission switching condition-setting menu.
Fig. 20 is a diagram illustrating that a mode is automatically switched to the multi-light emission mode from the mono-light emission mode.
Fig. 21 is a diagram illustrating that a mode is automatically switched to the multi-light emission mode from the mono-light emission mode in a case where an observation portion returns to the original observation portion and the original observation portion is observed.
Fig. 22 is a diagram illustrating that a mode is automatically switched to the multi-light emission mode from the mono-light emission mode in a case where brightness is equal to or higher than the first brightness threshold value or brightness is equal to or lower than the second brightness threshold value.
Fig. 23 is a diagram illustrating that a mode is automatically switched to the multi-light emission mode from the mono-light emission mode in a case where the variation of magnification is less than the magnification threshold value.
Fig. 24 is a diagram illustrating that a mode is automatically switched to the multi-light emission mode from the mono-light emission mode in a case where the variation of an observation distance is equal to or smaller than the distance threshold value.
Fig. 25 is a diagram illustrating that a mode is automatically switched to the multi-light emission mode from the mono-light emission mode in a case where a shake amount is equal to or smaller than the shake-amount threshold value.
Fig. 26 is a flowchart showing a series of flows of the present invention.

### DESCRIPTION OF THE PREFERRED EMBODIMENTS

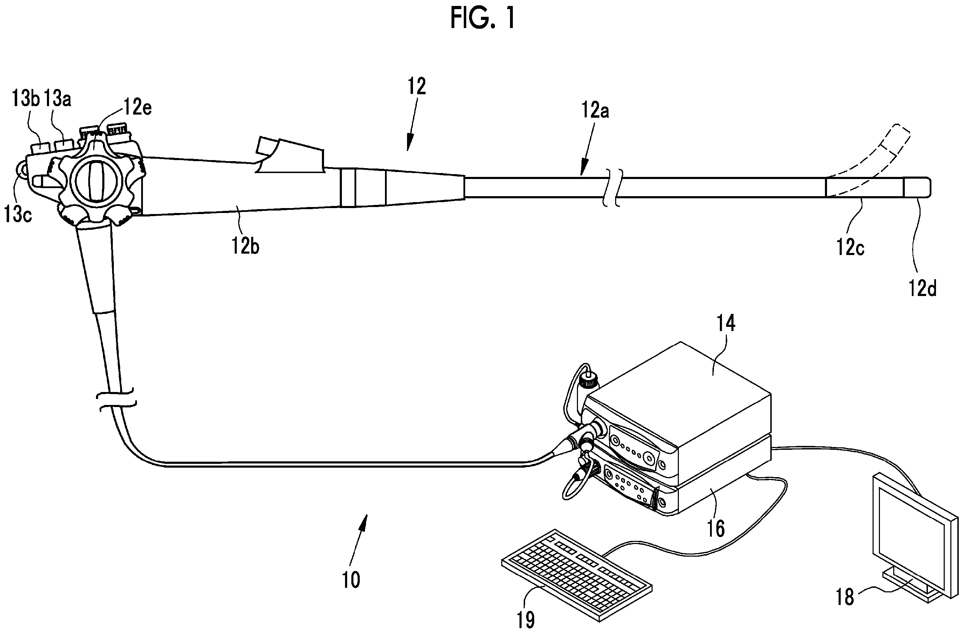

As shown in Fig. 1, an endoscope system 10 includes an endoscope 12, a light source device 14, a processor device 16, a display 18, and a user interface 19. The endoscope 12 is optically connected to the light source device 14, and is electrically connected to the processor device 16. The endoscope 12 includes an insertion part 12a that is to be inserted into an object to be examined, an operation part 12b that is provided at the proximal end portion of the insertion part 12a, and a bendable part 12c and a distal end part 12d that are provided on the distal end side of the insertion part 12a. In a case where angle knobs 12e of the operation part 12b are operated, the bendable part 12c is operated to be bent. As the bendable part 12c is operated to be bent, the distal end part 12d faces in a desired direction. The user interface 19 includes a mouse and the like in addition to a keyboard shown in Fig. 1.

Further, the operation part 12b is provided with a mode changeover SW 13a, a static-image-acquisition instruction part 13b, and a zoom operation part 13c in addition to the angle knobs 12e. The mode changeover SW 13a is used to switch a mono-light emission mode and a multi-light emission mode. In the mono-light emission mode, normal light (specific illumination light) is emitted and a normal image (specific observation image) is displayed on the display 18. In the multi-light emission mode, first illumination light for enhancing superficial blood vessels and second illumination light for enhancing deep blood vessels are emitted while being switched according to a specific light emission pattern. Furthermore, in the multi-light emission mode, a first observation image, which is obtained in a case where an object to be observed is illuminated with the first illumination light, and a second observation image, which is obtained in a case where the object to be observed is illuminated with the second illumination light, are displayed on the display 18 while being switched according to a specific display pattern. In the mono-light emission mode, any of the first illumination light or the second illumination light may be emitted instead of the normal light.

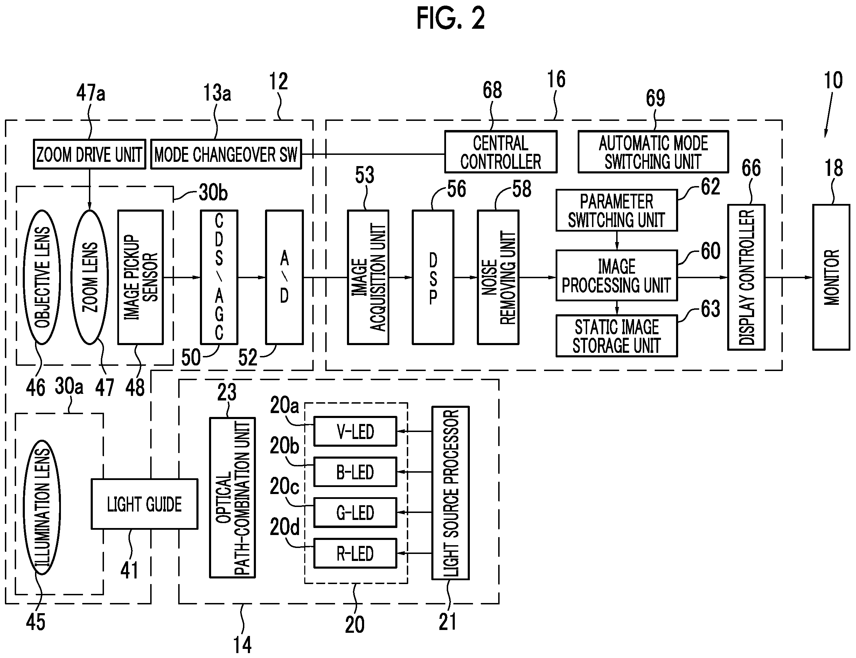

The static-image-acquisition instruction part 13b is used to instruct a static image storage unit 63 (see Fig. 2) to store the static image of the object to be observed. The zoom operation part 13c is used to operate a zoom lens 47 and a zoom drive unit 47a (see Fig. 2) that are provided in the endoscope 12.

The processor device 16 is electrically connected to the display 18 and the user interface 19. The display 18 outputs and displays image information and the like. The user interface 19 functions as a user interface (UI) that receives an input operation, such as function settings. An external recording unit (not shown), which records image information and the like, may be connected to the processor device 16.

As shown in Fig. 2, the light source device 14 includes a light source unit 20, a light source processor 21, and an optical path-combination unit 23. The light source unit 20 can emit light having a plurality of wavelength ranges, and can change the light emission ratio of the light having each wavelength range. In this specification, "light having a plurality of wavelength ranges different from each other" means that the plurality of wavelength ranges may partially overlap with each other without meaning that the plurality of wavelength ranges do not overlap with each other at all. The light source unit 20 includes a violet light emitting diode (V-LED) 20a, a blue light emitting diode (B-LED) 20b, a green light emitting diode (G-LED) 20c, and a red light emitting diode (R-LED) 20d in order to emit light having a plurality of wavelength ranges. Since the light source unit 20 may be provided with a plurality of semiconductor light sources, a laser diode (LD) may be used instead of the LED.

The light source processor 21 controls the drive of the LEDs 20a to 20d. The optical path-combination unit 23 combines the optical paths of four types of color light that are emitted from the four color LEDs 20a to 20d. The inside of an object to be examined is irradiated with the pieces of light, which are combined by the optical path-combination unit 23, through a light guide 41 inserted into the insertion part 12a and an illumination lens 45.

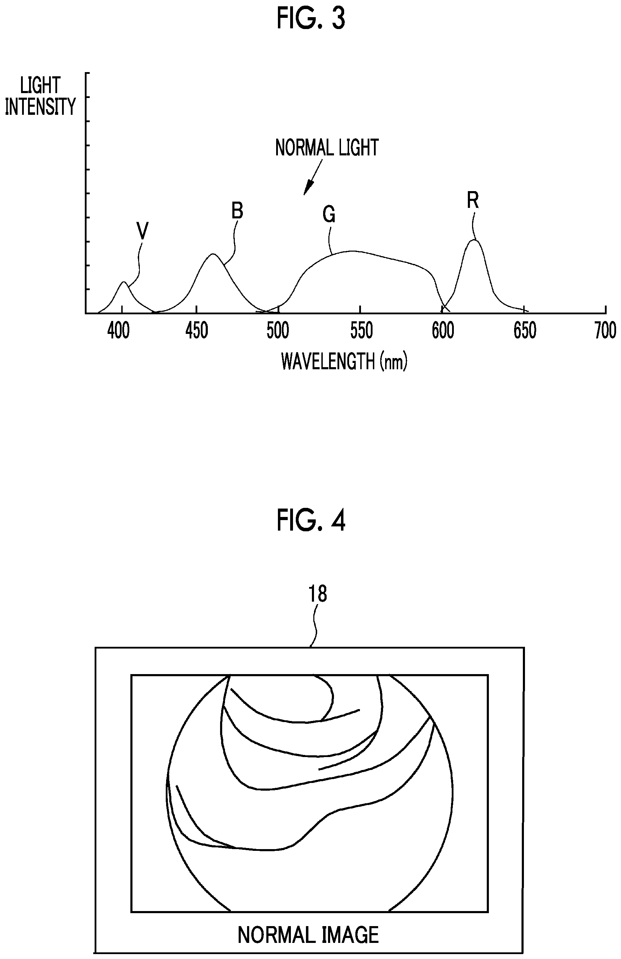

As shown in Fig. 3, the V-LED 20a generates violet light V of which the central wavelength is in a range of 405±10 nm and the wavelength range is in a range of 380 to 420 nm. The B-LED 20b generates blue light B of which the central wavelength is in a range of 460±10 nm and the wavelength range is in a range of 420 to 500 nm. The G-LED 20c generates green light G of which the wavelength range is in a range of 480 to 600 nm. The R-LED 20d generates red light R of which the central wavelength is in a range of 620 to 630 nm and the wavelength range is in a range of 600 to 650 nm.

The light source processor 21 performs a control to turn on the V-LED 20a, the B-LED 20b, the G-LED 20c, and the R-LED 20d in all observation modes. Further, the light source processor 21 controls the respective LEDs 20a to 20d so that normal light of which a light intensity ratio between violet light V, blue light B, green light G, and red light R is Vc:Bc:Gc:Rc is emitted in the mono-light emission mode (see Fig. 3). In a case where the image of the object to be observed illuminated with this normal light is picked up, the first observation image in which superficial blood vessels are enhanced as shown in Fig. 4 is obtained. In this specification, a light emission ratio means a light intensity ratio between the respective semiconductor light sources and includes a case where the light intensity ratio is 0 (zero). Accordingly, the light intensity ratio includes a case where any one or two or more of the respective semiconductor light sources are not turned on. For example, even though only one semiconductor light source is turned on and the other three semiconductor light sources are not turned on as in a case where a light intensity ratio between violet light V, blue light B, green light G, and red light R is 1:0:0:0, it is regarded that the light source unit 20 has a light emission ratio.

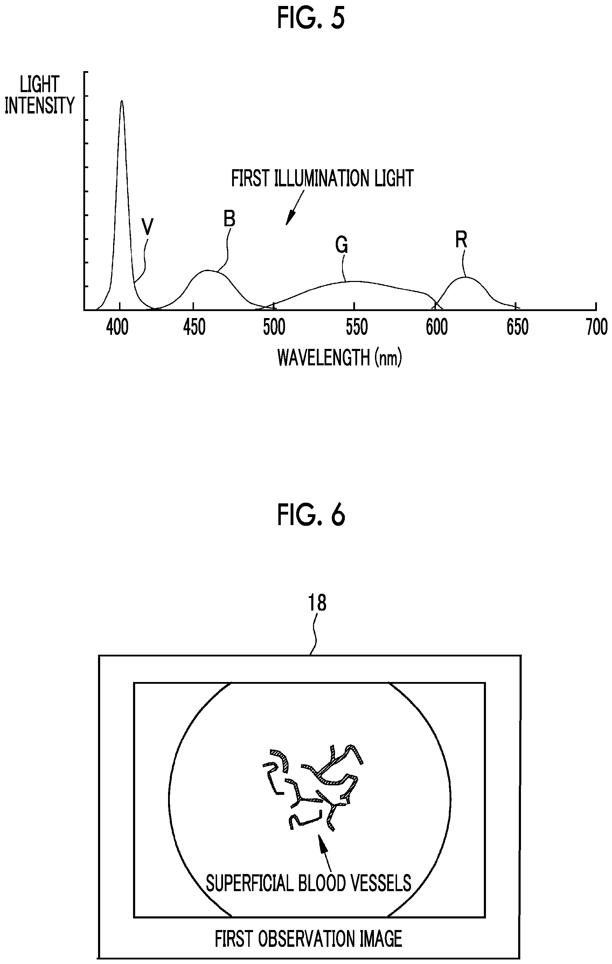

Furthermore, the light source processor 21 controls the respective LEDs 20a to 20d so that the first illumination light to be emitted in the case of the multi-light emission mode is emitted to have a light emission ratio between violet light V, blue light B, green light G, and red light R of Vs1:Bs1:Gs1:Rs1. It is preferable that the first illumination light has a peak in a range of 400 nm to 440 nm. For this purpose, Vs1:Bs1:Gs1:Rs1 of the first illumination light is set so that the light intensity of violet light V is higher than the light intensity of each of blue light B, green light G, and red light R as shown in Fig. 5 (Vs1>Bs1, Gs1, and Rs1). In a case where the image of the object to be observed illuminated with the first illumination light is picked up, the first observation image in which superficial blood vessels are enhanced as shown in Fig. 6 is obtained.

Further, since the first illumination light includes a first red-light wavelength range like red light R, the first illumination light can accurately reproduce the color of a mucous membrane. Furthermore, since the first illumination light includes a first blue-light wavelength range and a first green-light wavelength range like violet light V, blue light B, and green light G, the first illumination light can also enhance various structures, such as glandular structures and uneven portions, in addition to the above-mentioned superficial blood vessels.

Furthermore, the light source processor 21 controls the respective LEDs 20a to 20d so that the second illumination light to be emitted in the case of the multi-light emission mode is emitted to have a light emission ratio between violet light V, blue light B, green light G, and red light R of Vs2:Bs2:Gs2:Rs2. It is preferable that the intensity ratio of the second illumination light is higher than that of the first illumination light at wavelengths of 460 nm, 540 nm, and 630 nm.

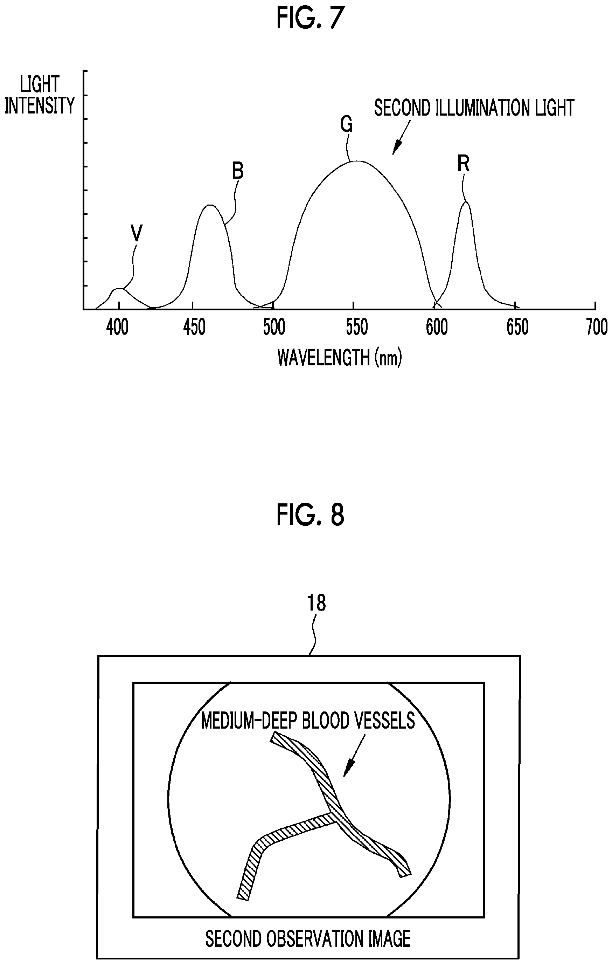

For this purpose, Vs2:Bs2:Gs2:Rs2 of the second illumination light is set so that the amounts of blue light B, green light G, and red light R of the second illumination light are larger than the amounts of blue light B, green light G, and red light R of the first illumination light as shown in Fig. 7. In a case where the image of the object to be observed illuminated with the second illumination light is picked up, the first observation image in which medium-deep blood vessels are enhanced as shown in Fig. 8 is obtained.

Vs2:Bs2:Gs2:Rs2 is set so that the light intensity of violet light V is lower than the light intensity of each of blue light B, green light G, and red light R (Vs2<Bs2, Gs2, and Rs2). Further, since the second illumination light includes a second red-light wavelength range like red light R, the second illumination light can accurately reproduce the color of a mucous membrane. Furthermore, since the second illumination light includes a second blue-light wavelength range and a second green-light wavelength range like violet light V, blue light B, and green light G, the second illumination light can also enhance various structures, such as glandular structures and uneven portions, in addition to the above-mentioned deep blood vessels.

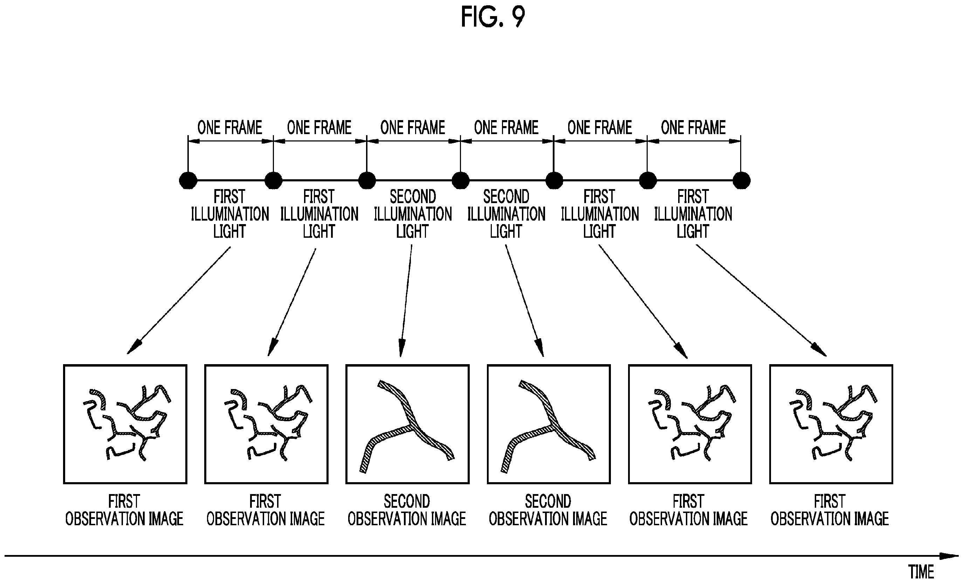

Further, in a case where a mode is set to the multi-light emission mode, the light source processor 21 performs a control to emit the first illumination light and the second illumination light while automatically switching the first illumination light and the second illumination light according to the specific light emission pattern. In this embodiment, as the specific light emission pattern, the first illumination light and the second illumination light are emitted while being switched at an interval of two frames as shown in Fig. 9. Further, as the specific display pattern, the first observation image obtained using the light emission of the first illumination light and the second observation image obtained using the light emission of the second illumination light are displayed on the display 18 while being switched at an interval of two frames.

As shown in Fig. 2, the light guide 41 is built in the endoscope 12 and a universal cord (a cord connecting the endoscope 12 to the light source device 14 and the processor device 16), and transmits the pieces of light, which are combined by the optical path-combination unit 23, to the distal end part 12d of the endoscope 12. A multimode fiber can be used as the light guide 41. For example, a thin fiber cable of which a total diameter of a core diameter of 105 µm, a cladding diameter of 125 µm, and a protective layer forming a covering is in a range of ϕ 0.3 to 0.5 mm can be used.

The distal end part 12d of the endoscope 12 is provided with an illumination optical system 30a and an image pickup optical system 30b. The illumination optical system 30a includes an illumination lens 45, and an object to be observed is irradiated with light transmitted from the light guide 41 through the illumination lens 45. The image pickup optical system 30b includes an objective lens 46, a zoom lens 47, and an image pickup sensor 48. Light reflected from the object to be observed is incident on the image pickup sensor 48 through the objective lens 46 and the zoom lens 47. Accordingly, the reflected image of the object to be observed is formed on the image pickup sensor 48. The zoom lens 47 can be moved along an optical axis by the zoom drive unit 47a. The size of the image of the object to be observed is increased or reduced depending on the movement of the zoom lens 47. A "magnification change unit" of the present invention corresponds to a configuration that includes the zoom operation part 13c, the zoom lens 47, and the zoom drive unit 47a.

The image pickup sensor 48 is a color image pickup sensor, and picks up the reflected image of an object to be examined and outputs image signals. It is preferable that the image pickup sensor 48 is a charge coupled device (CCD) image pickup sensor, a complementary metal-oxide semiconductor (CMOS) image pickup sensor, or the like. The image pickup sensor 48 used in the present invention is a color image pickup sensor that is used to obtain RGB image signals corresponding to three colors of R (red), G (green), and B (blue), that is, a so-called RGB image pickup sensor that comprises R-pixels provided with R-filters, G-pixels provided with G-filters, and B-pixels provided with B-filters.

The image pickup sensor 48 may be a so-called complementary color image pickup sensor, which comprises complementary color filters corresponding to C (cyan), M (magenta), Y (yellow), and G (green), instead of an RGB color image pickup sensor. In a case where a complementary color image pickup sensor is used, image signals corresponding to four colors of C, M, Y, and G are output. Accordingly, the image signals corresponding to four colors of C, M, Y, and G need to be converted into image signals corresponding to three colors of R, G, and B by complementary color-primary color conversion. Further, the image pickup sensor 48 may be a monochrome image pickup sensor that includes no color filter. In this case, the light source processor 21 needs to cause blue light B, green light G, and red light R to be emitted in a time-sharing manner, and add demosaicing to the processing of image pickup signals.

The image signals output from the image pickup sensor 48 are transmitted to a CDS/AGC circuit 50. The CDS/AGC circuit 50 performs correlated double sampling (CDS) or auto gain control (AGC) on the image signals that are analog signals. The image signals, which have been transmitted through the CDS/AGC circuit 50, are converted into digital image signals by an analog/digital converter (A/D converter) 52. The digital image signals, which have been subjected to A/D conversion, are input to the processor device 16.

In the processor device 16, a program related to processing, such as automatic mode switching, is stored in a program memory (not shown). In the processor device 16, the program stored in the program memory is executed by a central controller 68 formed of an image control processor, so that the functions of an image acquisition unit 53, a digital signal processor (DSP) 56, a noise removing unit 58, an image processing unit 60, a parameter switching unit 62, a display controller 66, and an automatic mode switching unit 69 are realized.

Digital color image signals output from the endoscope 12 are input to the image acquisition unit 53. The color image signals are RGB image signals that are formed of R-image signals output from the R-pixels of the image pickup sensor 48, G-image signals output from the G-pixels of the image pickup sensor 48, and B-image signals output from the B-pixels of the image pickup sensor 48.

The DSP 56 performs various types of signal processing, such as defect correction processing, offset processing, gain processing, color adjustment processing, gamma conversion processing, and demosaicing processing, on the received image signals. Signals of defective pixels of the image pickup sensor 48 are corrected in the defect correction processing. Dark current components are removed from the RGB image signals having been subjected to the defect correction processing in the offset processing, so that an accurate zero level is set.

The RGB image signals having been subjected to the offset processing are multiplied by a specific gain parameter in the gain processing, so that signal levels are adjusted. The specific gain parameter varies for each observation mode. For example, gain processing for normal light for multiplying image signals, which are obtained from the illumination of the normal light and image pickup, by a gain parameter for normal light as the specific gain parameter is performed in the case of the mono-light emission mode. Further, in the case of the multi-light emission mode, gain processing for first illumination light for multiplying RGB image signals, which are obtained from the illumination of the first illumination light and image pickup, by a gain parameter for first illumination light as the specific gain parameter is performed at the time of the illumination of the first illumination light, and gain processing for second illumination light for multiplying RGB image signals, which are obtained from the illumination of the second illumination light and image pickup, by a gain parameter for second illumination light as the specific gain parameter is performed at the time of the illumination of the second illumination light.

After that, brightness or a chroma saturation is adjusted by the gamma conversion processing. The demosaicing processing (also referred to as equalization processing or demosaicing) is performed on the RGB image signals having been subjected to linear matrix processing, so that signals of colors deficient in each pixel are generated by interpolation. All the pixels are made to have the signals of the respective colors of R, G, and B by this demosaicing processing.

The noise removing unit 58 performs noise removal processing (for example, a moving-average method, a median filtering method, or the like) on the RGB image signals, which have been subjected to gamma correction and the like by the DSP 56, to remove noise from the RGB image signals. The RGB image signals from which noise has been removed are transmitted to the image processing unit 60.

The image processing unit 60 performs various types of image processing on the RGB image signals. The various types of image processing include image processing that is performed under a condition varying for each mode in addition to image processing that is performed under the same condition regardless of the mono-light emission mode or the multi-light emission mode. The image processing that is performed under a condition varying for each mode includes color adjustment processing for improving color reproducibility and structure enhancement processing for enhancing various structures, such as blood vessels and uneven portions. Each of the color adjustment processing and the structure enhancement processing is processing that uses a two-dimensional look up table (LUT), a three-dimensional look up table (LUT), a matrix, or the like. In a case where the color enhancement processing and the structure enhancement processing are to be performed, a color enhancement processing parameter and a structure enhancement processing parameter set for each observation mode are used in the image processing unit 60. The switching of the color enhancement processing parameter or the structure enhancement processing parameter is performed by the parameter switching unit 62.

In a case where a mode is set to the mono-light emission mode, a parameter to be used in the image processing unit 60 is switched to a color enhancement processing parameter for normal light and a structure enhancement processing parameter for normal light by the parameter switching unit 62. Then, the image processing unit 60 performs color enhancement processing for normal light on the RGB image signals using the color enhancement processing parameter for normal light, and performs structure enhancement processing for normal light on the RGB image signals using the structure enhancement processing parameter for normal light. After that, the RGB image signals having been subjected to the above-mentioned processing are input to the display controller 66 as the normal image.

In a case where a mode is set to the multi-light emission mode, the image processing unit 60 performs color enhancement processing for first illumination light and structure enhancement processing for first illumination light on the RGB image signals at the time of the illumination of the first illumination light. After that, the RGB image signals having been subjected to the above-mentioned processing are input to the display controller 66 as the first observation image. Further, the image processing unit 60 performs color enhancement processing for second illumination light and structure enhancement processing for second illumination light on the RGB image signals at the time of the illumination of the second illumination light. Furthermore, in a case where a mode is set to the multi-light emission mode, the image processing unit 60 performs mucous membrane-color-balance processing for setting the colors of normal mucous membranes, which are included in the object to be observed, to the same color between the first observation image and the second observation image. First mucous membrane-color-balance processing is performed on the first observation image, and second mucous membrane-color-balance processing based on the result of the first mucous membrane-color-balance processing is performed on the second observation image. After that, the RGB image signals having been subjected to the above-mentioned processing are input to the display controller 66 as the second observation image.

B1-image signals, G1-image signals, and R1-image signals included in the first observation image are automatically adjusted in the first mucous membrane-color-balance processing as described in, for example, D1) to D3) to be described below so that the average color of the entire screen has a specific color balance. The first mucous membrane-color-balance processing is performed on the assumption that the color of a mucous membrane is dominant over the object to be observed. Then, the first mucous membrane-color-balance processing is performed, so that B1^{∗}-image signals, G1^{∗}-image signals, and R1^{∗}-image signals having been subjected to the first mucous membrane-color-balance processing are obtained.
D1) B1^{∗}-image signal=B1/B1ave
D2) G1^{∗}-image signal=G1/G1ave
D3) R1^{∗}-image signal=R1/R1ave

Here, Blave denotes the average pixel value of the B1-image signals (the sum of pixel values of the entire screen (effective pixels)/the number of effective pixels). Glave denotes the average pixel value of the G1-image signals (the sum of pixel values of the entire screen (effective pixels)/the number of effective pixels). Rlave denotes the average pixel value of the R1-image signals (the sum of pixel values of the entire screen (effective pixels)/the number of effective pixels).

Further, B2-image signals, G2-image signals, and R2-image signals included in the second observation image are automatically adjusted in the second mucous membrane-color-balance processing as described in, for example, E1) to E3) to be described below so that the average color of the entire screen has a specific color balance. B1ave, G1ave, and Rlave calculated in the first mucous membrane-color-balance processing are used in the second mucous membrane-color-balance processing. Then, the second mucous membrane-color-balance processing is performed, so that B2^{∗}-image signals, G2^{∗}-image signals, and R2^{∗}-image signals having been subjected to the second mucous membrane-color-balance processing are obtained.
E1) B2^{∗}-image signal=B2/B1ave
E2) G2^{∗}-image signal=G2/G1ave
E3) R2^{∗}-image signal=R2/R1ave

The display controller 66 performs a control to display the normal image, the first observation image, or the second observation image, which is input from the image processing unit 60, as an image that can be displayed on the display 18. In the case of the mono-light emission mode, the display controller 66 causes the display 18 to display the normal image. In the case of the multi-light emission mode, the display controller 66 causes the display 18 to display the first observation image or the second observation image while switching the first observation image and the second observation image according to a specific display pattern (in this embodiment, at an interval of two frames. See Fig. 9).

The central controller 68 controls the respective parts of the processor device 16 in addition to executing the program as described above. Further, the central controller 68 receives information from the endoscope 12 or the light source device 14, and performs the control of the respective parts of the processor device 16 and the control of the endoscope 12 or the light source device 14 on the basis of the received information.

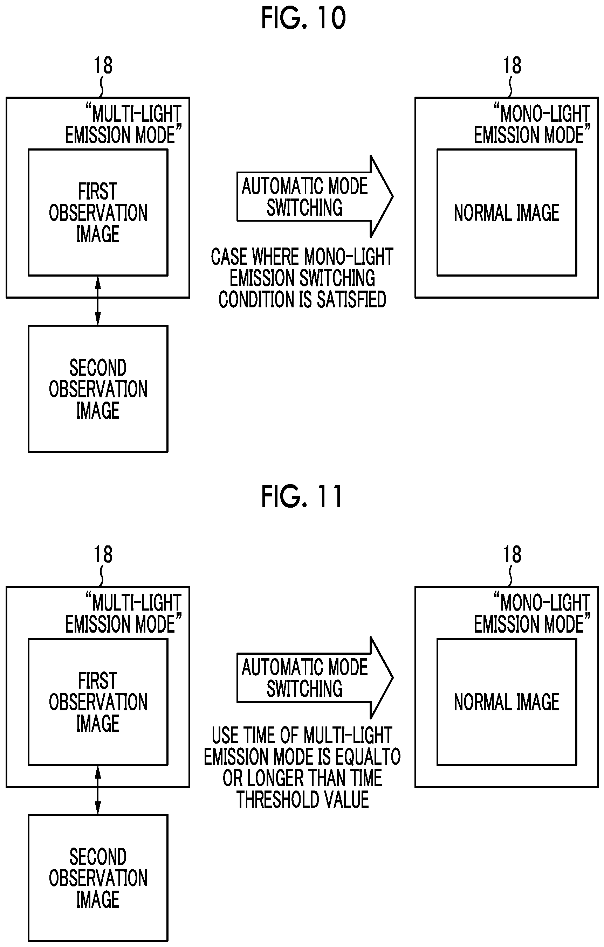

In a case where a mode is set to the multi-light emission mode and a preset mono-light emission switching condition is satisfied, as shown in Fig. 10, the automatic mode switching unit 69 performs processing for switching a mode to the mono-light emission mode where only the normal image continues to be displayed on the display 18 from the multi-light emission mode where the first observation mode and the second observation mode are displayed on the display 18 while being switched. The reason why a mode is adapted to be capable of being automatically switched is that a user forgets to switch a mode in a case where the object to be observed is not changed or is hardly changed. Since there is a case where it is difficult for a user to know to which one of the multi-light emission mode and the mono-light emission mode a mode is set, the display 18 displays that a mode is set to the "multi-light emission mode" in the case of the multi-light emission mode and displays that a mode is set to the "mono-light emission mode" in the case of the mono-light emission mode.

The mono-light emission switching condition, which is used to switch a mode to the mono-light emission mode from the multi-light emission mode, is that, for example, the use time of the multi-light emission mode is equal to or longer than a predetermined time threshold value. In this case, a time, which has passed after a mode is set to the multi-light emission mode by the mode changeover SW 13a, is measured by a time measurement unit (not shown) provided in the processor device 16. Then, in a case where the measured time is equal to or longer than the time threshold value, a mode is automatically switched to the mono-light emission mode from the multi-light emission mode as shown in Fig. 11.

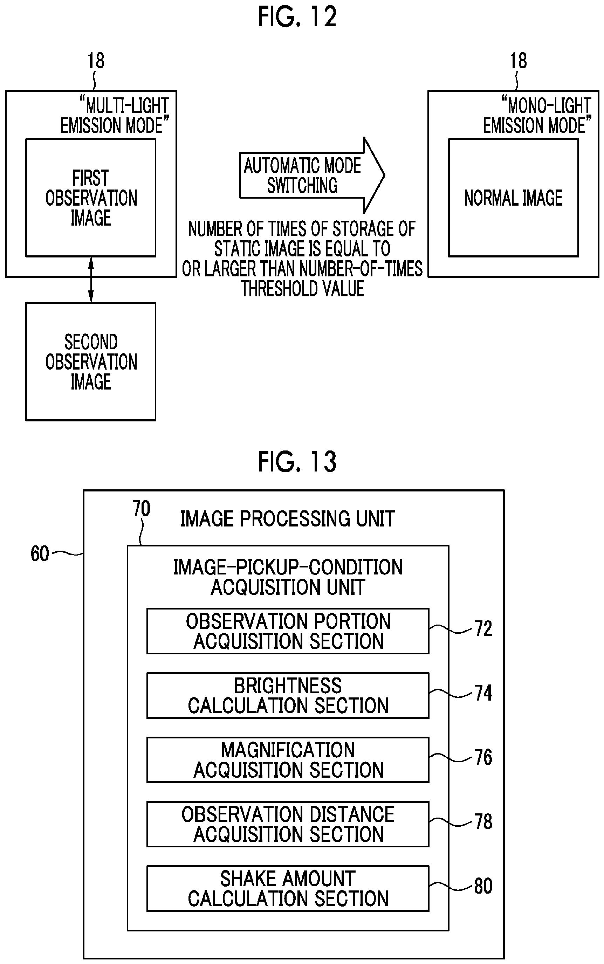

Further, the mono-light emission switching condition is that, for example, the number of times of storage of the static image of the object to be observed is equal to or larger than a predetermined number-of-times threshold value is used. In this case, the number of times of operation of the static-image-acquisition instruction part 13b is counted by a number-of-times counter unit (not shown) provided in the processor device 16. Then, in a case where the counted number of times is equal to or larger than the number-of-times threshold value, a mode is automatically switched to the mono-light emission mode from the multi-light emission mode as shown in Fig. 12.

Furthermore, the mono-light emission switching condition is that image pickup conditions about the object to be observed are changed. In this case, an image-pickup-condition acquisition unit 70 is provided in the image processing unit 60 of the processor device 16 to acquire the image pickup conditions. As shown in Fig. 13, the image-pickup-condition acquisition unit 70 comprises an observation portion acquisition section 72, a brightness calculation section 74, a magnification acquisition section 76, an observation distance acquisition section 78, and a shake amount calculation section 80.

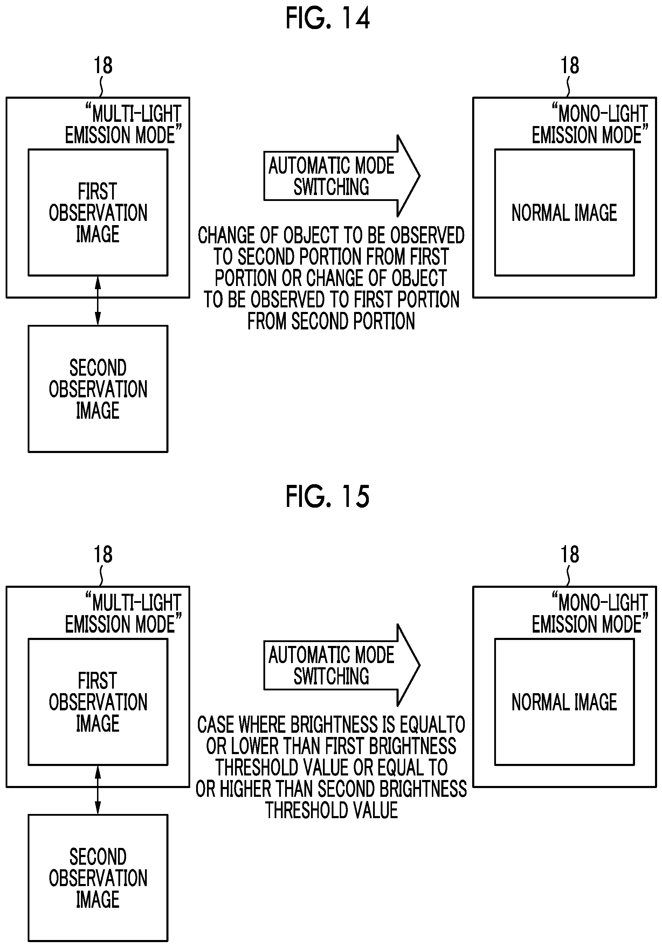

In a case where the mono-light emission switching condition is that an observation portion, which is one of the image pickup conditions, is changed, for example, an observation portion of which the image is picked up at present is changed to a second portion (for example, "stomach") from a first portion (for example, "gullet") or the observation portion is changed to the first portion from the second portion, a mode is automatically switched to the mono-light emission mode from the multi-light emission mode as shown in Fig. 14. Since it is thought that the distal end part 12d of the endoscope is moved in a case where the observation portion is changed, the object to be observed may not be reliably illuminated with the first illumination light and the second illumination light that are alternately emitted. For this reason, such a case is not suitable for the multi-light emission mode.

Information about the observation portion is acquired by the observation portion acquisition section 72. The observation portion acquisition section 72 determines the observation portion from the image feature quantity of the first observation image or the second observation image obtained in the multi-light emission mode. For example, in a case where the brightness of the central portion of the screen is lower than that of a peripheral portion thereof in the first observation image or the second observation image, the observation portion is determined as the "gullet". Further, in a case where the brightness of the central portion of the screen is higher than that of a peripheral portion thereof in the first observation image or the second observation image, the observation portion is determined as the "stomach".

In a case where the mono-light emission switching condition is that the brightness of the object to be observed, which is one of the image pickup conditions, is equal to or lower than a first brightness threshold value or is equal to or higher than a second brightness threshold value larger than the first brightness threshold value, a mode is automatically switched to the mono-light emission mode from the multi-light emission mode as shown in Fig. 15. In a case where the brightness is equal to or lower than the first brightness threshold value, the entire object to be observed is dark. For this reason, such a case is not suitable for the multi-light emission mode. Likewise, in a case where the brightness is equal to or higher than the second brightness threshold value, the entire object to be observed is extremely bright as in a case where halation occurs, and the like. For this reason, such a case is not suitable for the multi-light emission mode. Information about the brightness is acquired by the brightness calculation section 74. The brightness calculation section 74 calculates the average value of pixel values from the first observation image or the second observation image, and calculates brightness from the calculated average value of pixel values. Here, as the average value of pixel values is larger, brightness is higher.

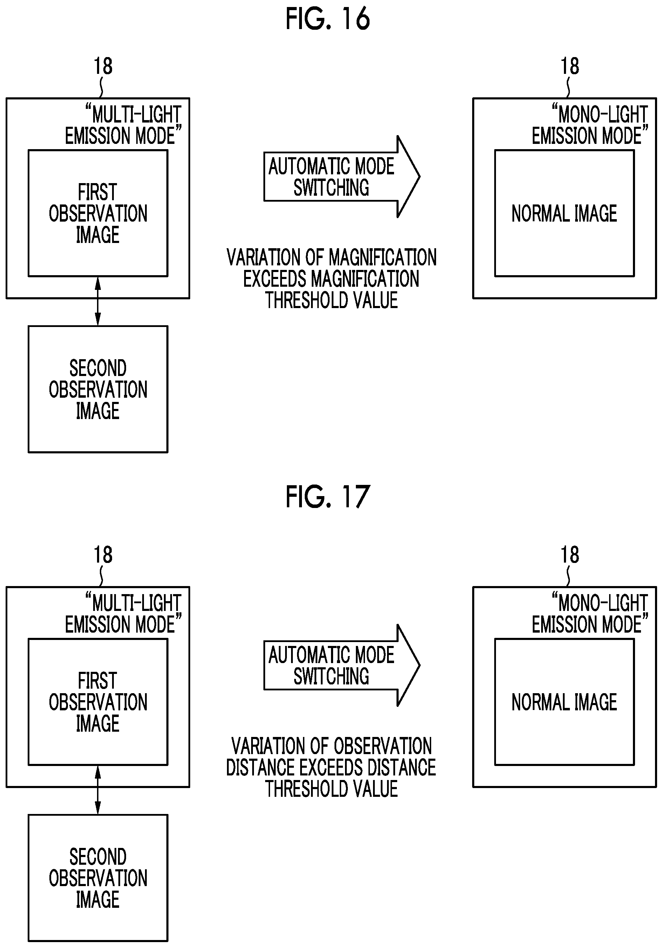

In a case where the mono-light emission switching condition is that the variation of the magnification of the object to be observed, which is one of the image pickup conditions, exceeds a magnification threshold value, a mode is automatically switched to the mono-light emission mode from the multi-light emission mode as shown in Fig. 16. In a case where the magnification of the object to be observed is significantly changed so that the variation of magnification exceeds the magnification threshold value, the illumination distribution of illumination light on the object to be observed is changed. For this reason, such a case is often not suitable for the multi-light emission mode. With regard to the magnification of the object to be observed, zoom information representing which the magnification of the object to be observed is set to is transmitted to the magnification acquisition section 76 whenever the zoom operation part 13c is operated. The automatic mode switching unit 69 determines whether or not the variation of magnification exceeds the magnification threshold value with reference to the zoom information acquired by the magnification acquisition section 76.

In a case where the mono-light emission switching condition is that the variation of an observation distance (a distance between the distal end part 12d of the endoscope and the object to be observed), which is one of the image pickup conditions, exceeds a distance threshold value, a mode is automatically switched to the mono-light emission mode from the multi-light emission mode as shown in Fig. 17. As with the magnification of the object to be observed, in a case where the observation distance is significantly changed so that the variation of the observation distance exceeds the distance threshold value, the illumination distribution of illumination light on the object to be observed is changed. For this reason, such a case is often not suitable for the multi-light emission mode. With regard to the observation distance, the observation distance acquisition section 78 calculates the average value of pixel values from the first observation image or the second observation image obtained in the multi-light emission mode and obtains an observation distance from the calculated average value of pixel values. Here, as the average value of pixel values is larger, the observation distance is shorter.

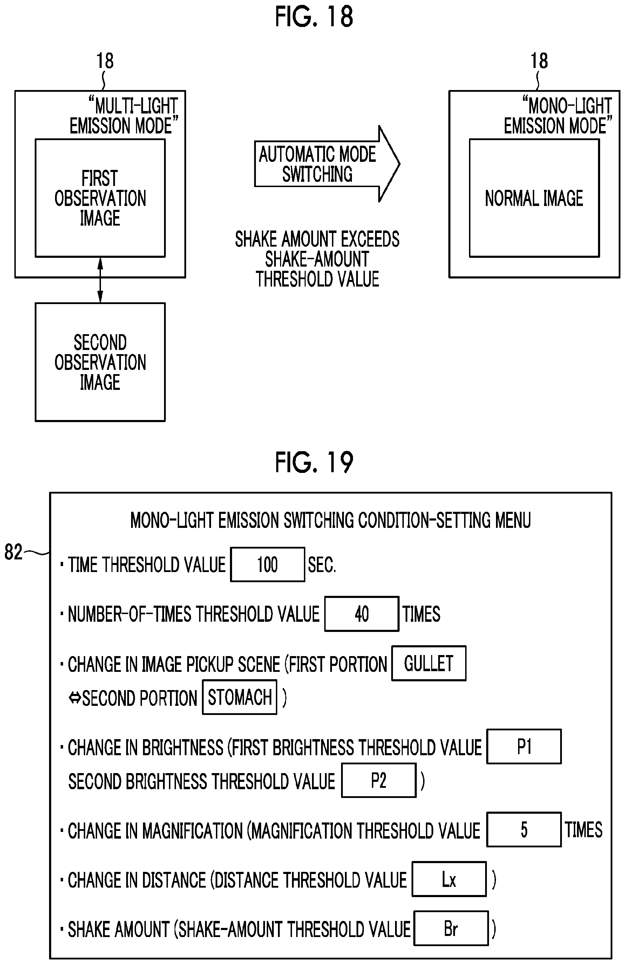

In a case where the mono-light emission switching condition is that the shake amount of the image, which is one of the image pickup conditions, exceeds a shake-amount threshold value, a mode is automatically switched to the mono-light emission mode from the multi-light emission mode as shown in Fig. 18. In a case where the shake amount of the image is large and exceeds the shake-amount threshold value, illumination light may not reliably reach the object to be observed. For this reason, such a case is often not suitable for the multi-light emission mode. With regard to the shake amount, the shake amount calculation section 80 obtains contrast from the first observation image or the second observation image obtained in the multi-light emission mode and calculates a shake amount from the contrast. As the contrast is lower, the shake amount is larger. A method of calculating the shake amount from the frequency component of the first observation image or the second observation image may be used as a method of calculating the shake amount in addition to a method using contrast (the shake amount is increased as the frequency component is a lower frequency component).

The mono-light emission switching condition that is used for the automatic switching of a mode to the mono-light emission mode from the multi-light emission mode can be appropriately set by a user as described above. In this case, a user operates the user interface 19 to cause the display 18 to display a mono-light emission switching condition-setting menu 82 shown in Fig. 19. The "time threshold value" and the "number-of-times threshold value" can be set in the mono-light emission switching condition-setting menu 82. In a case where the "time threshold value" is set to "100 sec." in the mono-light emission switching condition-setting menu 82, a mode is automatically switched to the mono-light emission mode from the multi-light emission mode at a point of time when the use time of the multi-light emission mode reaches "100 sec." Further, in a case where the "number-of-times threshold value" is set to "40 times" in the mono-light emission switching condition-setting menu 82, a mode is automatically switched to the mono-light emission mode from the multi-light emission mode at a point of time when the number of times of operation of the static-image-acquisition instruction part 13b reaches "40 times".

It is preferable that the same multi-light emission restart condition-setting menu as the mono-light emission switching condition-setting menu 82 is displayed on the display 18 so that a multi-light emission restart condition used for the automatic switching of a mode to the multi-light emission mode from the mono-light emission mode can be appropriately set as described later.

Further, the "first portion" and the "second portion" can be set in the mono-light emission switching condition-setting menu 82. In a case where the "first portion" is set to the "gullet" and the "second portion" is set to the "stomach" and the observation portion is changed to the "stomach" from the "gullet" or the observation portion is changed to the "gullet" from the "stomach", a mode is automatically switched to the mono-light emission mode from the multi-light emission mode. Furthermore, the "first brightness threshold value" and the "second brightness threshold value" can be set in the mono-light emission switching condition-setting menu 82. In a case where the "first brightness threshold value" is set to P1 and the "second brightness threshold value" is set to P2 (>P1) and the brightness of the object to be observed is equal to or lower than P1 or is equal to or higher than P2, a mode is automatically switched to the mono-light emission mode from the multi-light emission mode.

Further, the "magnification threshold value" can be set in the mono-light emission switching condition-setting menu 82. In a case where the "magnification threshold value" is set to "5 times", a mode is automatically switched to the mono-light emission mode from the multi-light emission mode at the point of time when the variation of magnification of the object to be observed exceeds "5 times". Furthermore, the "distance threshold value" can be set in the mono-light emission switching condition-setting menu 82. In a case where the "distance threshold value" is set to Lx and the variation of the observation distance exceeds Lx, a mode is automatically switched to the mono-light emission mode from the multi-light emission mode. Moreover, the "shake-amount threshold value" can be set in the mono-light emission switching condition-setting menu 82. In a case where the "shake-amount threshold value" is set to Br and the shake amount exceeds Br, a mode is automatically switched to the mono-light emission mode from the multi-light emission mode.

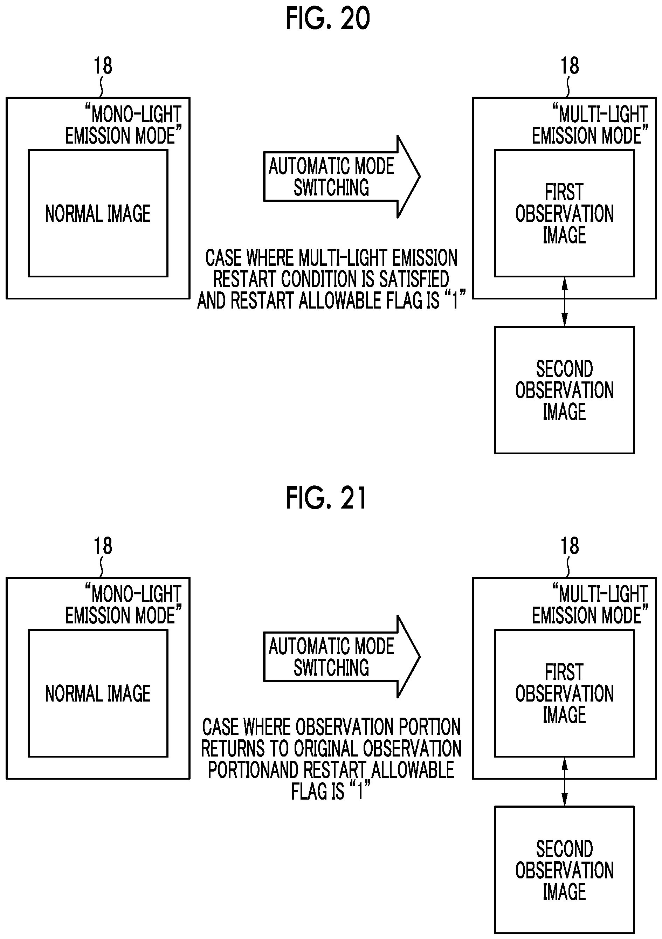

In a case where the mono-light emission switching condition is satisfied and a mode is thus automatically switched to the mono-light emission mode from the multi-light emission mode, the automatic mode switching unit 69 automatically switches a mode to the multi-light emission mode from the mono-light emission mode as shown in Fig. 20 in a case where a preset multi-light emission restart condition is satisfied. The automatic mode switching unit 69 automatically switches a mode to the mono-light emission mode in a case where the image pickup condition about the object to be observed is changed, and the like. However, it is preferable that the automatic mode switching unit 69 automatically switches a mode to the multi-light emission mode from the mono-light emission mode in a case where a change in the image pickup condition about the object to be observed is within an allowable range, and the like, that is, in a case where the multi-light emission restart condition is satisfied, and the like. "A change in the image pickup condition is within an allowable range" means that the visibility of the switching display of the first observation image and the second observation image obtained in the multi-light emission mode is not affected in spite of a change in the image pickup condition.

It is preferable that the multi-light emission restart condition is a case where the object to be observed returns to the first portion within a certain period of time after being changed to the second portion (for example, "stomach ") from the first portion (for example, "gullet") or a case where the object to be observed returns to the second portion within a certain period of time after being changed to the first portion from the second portion. In this case, a mode is automatically switched to the multi-light emission mode from the mono-light emission mode as shown in Fig. 21. The reason for this is that an illumination condition is not changed so much and a light emission state caused by the multi-light emission mode is not affected in a case where the observation portion immediately returns to the original portion. Information about the observation portion is acquired by the observation portion acquisition section 72 as described above.

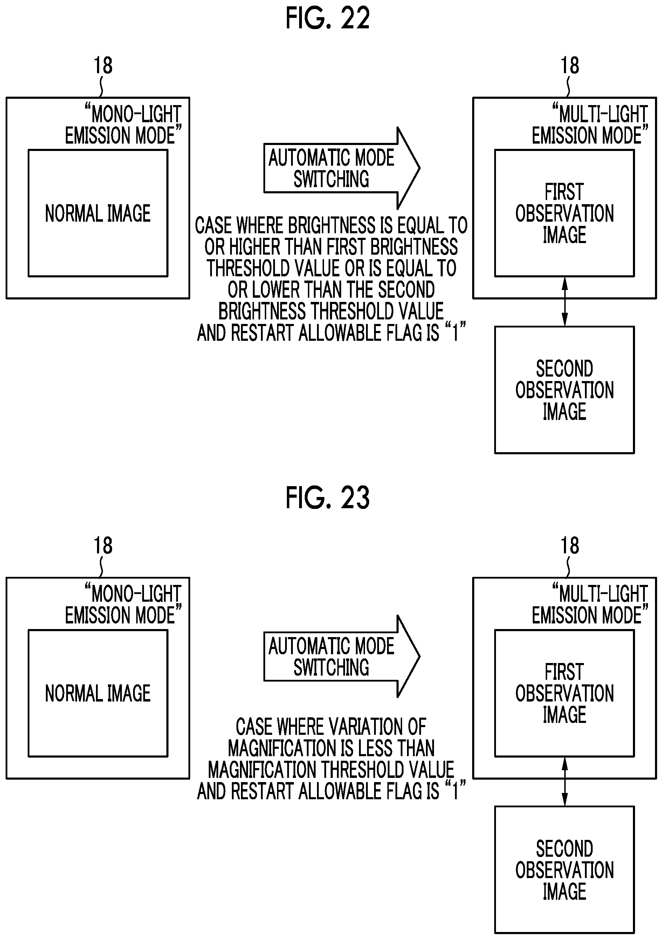

It is preferable that the multi-light emission restart condition is a case where the brightness of the object to be observed is equal to or higher than the first brightness threshold value or is equal to or lower than the first brightness threshold value. In this case, a mode is automatically switched to the multi-light emission mode from the mono-light emission mode as shown in Fig. 22. The reason for this is that a light emission state caused by the multi-light emission mode is not affected in a case where the brightness returns to original normal brightness (equal to or higher than the first brightness threshold value or equal to or lower than the first brightness threshold value) after a certain period of time even though the brightness is reduced or increased. Information about the brightness is acquired by the brightness calculation section 74 as described above.

It is preferable that the multi-light emission restart condition is that the variation of magnification of the object to be observed is less than the magnification threshold value in a case where the magnification of the object to be observed is changed. In this case, a mode is automatically switched to the multi-light emission mode from the mono-light emission mode as shown in Fig. 23. The reason for this is that a light emission state caused by the multi-light emission mode is not affected in a case where a change in the magnification of the object to be observed is not so large and the variation of magnification temporarily exceeds the magnification threshold value and immediately falls below the magnification threshold value. The magnification of the object to be observed is acquired by the magnification acquisition section 76 as described above.

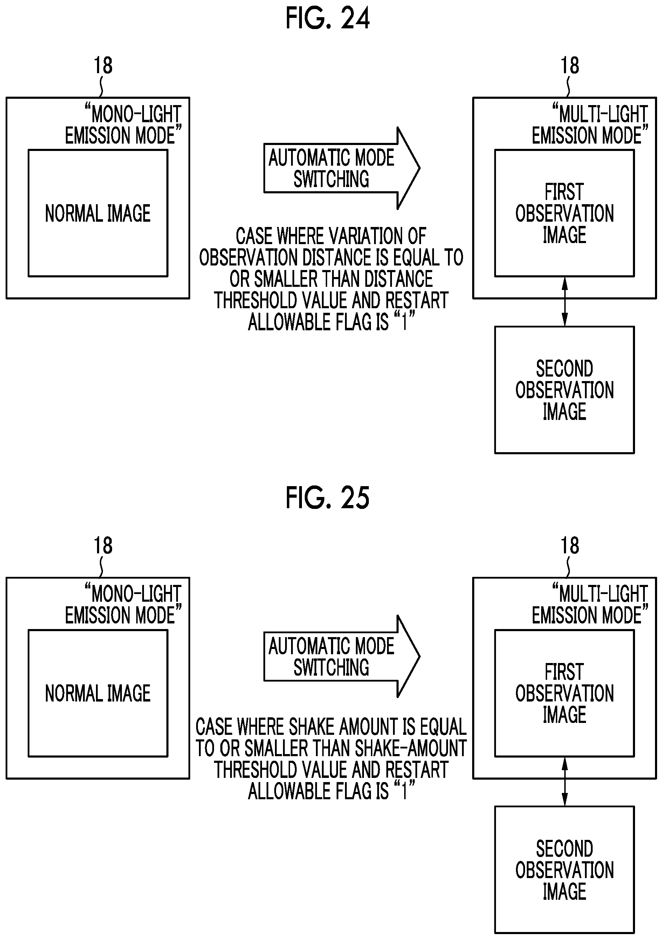

It is preferable that the multi-light emission restart condition is a case where the variation of an observation distance is equal to or smaller than the distance threshold value. In this case, a mode is automatically switched to the multi-light emission mode from the mono-light emission mode as shown in Fig. 24. The reason for this is that a light emission state caused by the multi-light emission mode is not affected in a case where a change in an observation distance is not so large and the variation of an observation distance temporarily exceeds the distance threshold value and immediately falls below the distance threshold value. An observation distance is acquired by the observation distance acquisition section 78 as described above.

It is preferable that the multi-light emission restart condition is a case where the shake amount of an observation image is equal to or smaller than the shake-amount threshold value. In this case, a mode is automatically switched to the multi-light emission mode from the mono-light emission mode as shown in Fig. 25. The reason for this is that a light emission state caused by the multi-light emission mode is not affected in a case where the shake amount of an image is not so large and the shake amount temporarily exceeds the shake-amount threshold value and immediately falls below the shake-amount threshold value. The shake amount is acquired by the shake amount calculation section 80 as described above.

However, in order to prevent a mode from being automatically switched to the multi-light emission mode unintentionally, it is preferable that the automatic mode switching unit 69 automatically switches a mode to the multi-light emission mode from the mono-light emission mode in a case where the multi-light emission restart condition is satisfied and a restart allowable condition is satisfied, but prohibits a mode from being automatically switched to the multi-light emission mode in a case where the multi-light emission restart condition is satisfied and the restart allowable condition is not satisfied. It is preferable that the restart allowable condition can be set by a user. Further, a restart allowable/unallowable flag representing the restart allowable condition is used in order to determine whether or not the restart allowable condition is satisfied. In a case where a restart allowable/unallowable flag is "1", it means that the restart allowable condition is not satisfied. In a case where a restart allowable/unallowable flag is "0", it means that the restart allowable condition is satisfied (see Figs. 20 to 25).

It is preferable that the restart allowable condition is a case where a user does not select the mono-light emission mode. It is preferable that a user operates the user interface 19 to select the mono-light emission mode. In a case where a user selects the mono-light emission mode, the restart allowable/unallowable flag is "0". In a case where a user does not select the mono-light emission mode, the restart allowable/unallowable flag is "1". It is preferable that a mode is not automatically switched to the multi-light emission mode in a case where a user voluntarily selects the mono-light emission mode.

It is preferable that the restart allowable condition is a case where a restart allowable time does not elapse yet after the mono-light emission switching condition is satisfied and a mode is automatically switched to the mono-light emission mode from the multi-light emission mode. In this case, a time elapsed after the switching of a mode to the mono-light emission mode is measured by the time measurement unit (not shown). In a case where the measured time is shorter than the restart allowable time, the restart allowable/unallowable flag is maintained at "1". Further, in a case where the measured time is equal to or longer than the restart allowable time, the restart allowable/unallowable flag is switched to "0". Since it is thought that a state after the restart allowable time has elapsed is suitable for an observation based on the mono-light emission mode, it is preferable that a mode is not automatically switched to the multi-light emission mode.

It is preferable that the restart allowable condition is a case where the multi-light emission mode is implementable. Whether the multi-light emission mode can be implemented or not is determined by a multi-light emission mode-enablement-determination unit (not shown) of the processor device 16. Specifically, the multi-light emission mode-enablement-determination unit detects abnormalities and the like related to the endoscope 12, the light source device 14, and the processor device 16, and determines whether the multi-light emission mode can be implemented or not on the basis of detection results. In this case, the restart allowable/unallowable flag is set to "1" in a case where the multi-light emission mode-enablement-determination unit determines that the multi-light emission mode can be implemented, and the restart allowable/unallowable flag is set to "0" in a case where the multi-light emission mode-enablement-determination unit determines that the multi-light emission mode cannot be implemented. In a case where abnormalities and the like related to the endoscope 12, the light source device 14, and the processor device 16 occur and it is difficult to implement the multi-light emission mode, it is preferable that a mode is not automatically switched to the multi-light emission mode.

It is preferable that the restart allowable condition is a case where the use or non-use of the magnification change unit is not switched. The use of the magnification change unit means a state where the zoom operation part 13c is set to "ON" so that a magnification can be changed. The non-use of the magnification change unit means a state where the zoom operation part 13c is set to "OFF" so that a magnification is not changed. In this case, after the mono-light emission switching condition is satisfied and a mode is automatically switched to the mono-light emission mode, the restart allowable/unallowable flag is set to "1" in a case where the use or non-use of the magnification change unit is not switched and the restart allowable/unallowable flag is set to "0" in a case where the use or non-use of the magnification change unit is switched. In a case where the use or non-use of the magnification change unit is switched, an object different from that in the case of the previous multi-light emission mode is observed and it is preferable that a mode is not automatically switched to the multi-light emission mode since the multi-light emission mode is not necessarily used.

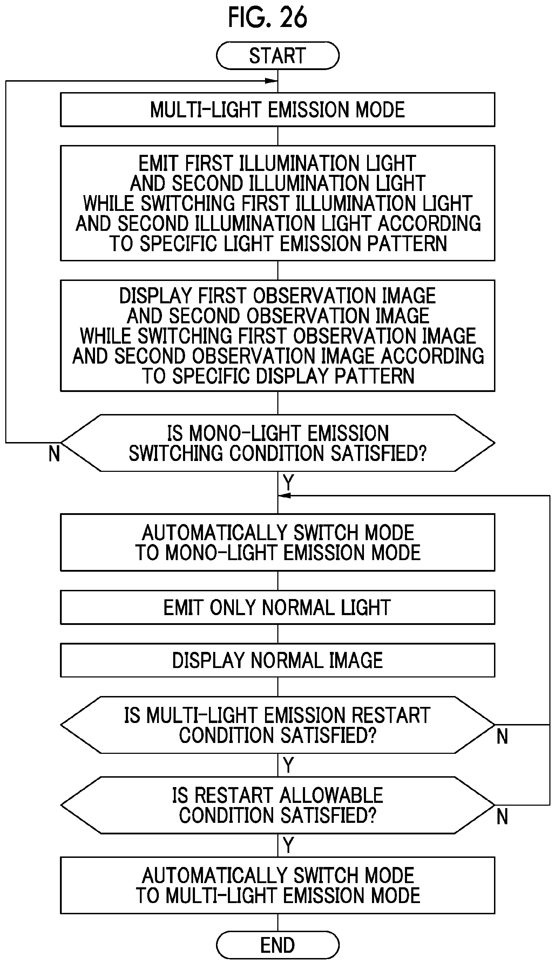

Next, a series of flows including the present invention will be described with reference to a flowchart shown in Fig. 26. In the multi-light emission mode, the first illumination light and the second illumination light are emitted while being switched according to a specific light emission pattern (in this embodiment, at an interval of two frames). Further, the first observation image obtained from the image pickup of an object to be observed illuminated with the first illumination light and the second observation image obtained from the image pickup of the object to be observed illuminated with the second illumination light are displayed on the display 18 while being switched according to a specific display pattern (in this embodiment, at an interval of two frames).

Then, in a case where a predetermined mono-light emission switching condition is satisfied, a mode is automatically switched to the mono-light emission mode from the multi-light emission mode. The mono-light emission switching condition includes a case where the use time of the multi-light emission mode exceeds the time threshold value, a case where the number of times of storage of a static image exceeds the number-of-times threshold value, and the like. Moreover, the mono-light emission switching condition includes a case where the image pickup conditions about the object to be observed are changed. In a case where a mode is automatically switched to the mono-light emission mode, the switching and emission of the first illumination light and the second illumination light are stopped. According to this, the switching and display of the first and second observation images are also stopped. Then, the normal light is emitted and the normal image obtained from the image pickup of the object to be observed illuminated with the normal light is displayed on the display 18.

After a mode is automatically switched to the mono-light emission mode, the processor device 16 monitors whether or not the multi-light emission restart condition is satisfied. In a case where the multi-light emission restart condition is satisfied and the restart allowable condition is satisfied, a mode is automatically switched to the multi-light emission mode from the mono-light emission mode. On the other hand, in a case where the restart allowable condition is not satisfied even though the multi-light emission restart condition is satisfied, the automatic switching of a mode to the multi-light emission mode is prohibited. Further, even in a case where the multi-light emission restart condition is not satisfied, the automatic switching of a mode to the multi-light emission mode is prohibited. In a case where the automatic switching of a mode to the multi-light emission mode is prohibited, the mono-light emission mode is continued.

In the embodiment, the first illumination light and the second illumination light have been emitted while being switched according to the specific light emission pattern, and the first observation image corresponding to the first illumination light and the second observation image corresponding to the second illumination light have been displayed on the display 18 while being switched according to the specific display pattern. However, three or more types of illumination light having wavelength ranges different from each other may be emitted while being switched according to a specific light emission pattern, and three or more types of observation images corresponding to the three or more types of illumination light may be displayed on the display 18 while being switched according to a specific display pattern.

The hardware structures of the processing units, which are included in the processor device 16 in the embodiment, such as the image acquisition unit 53, the DSP 56, the noise removing unit 58, the image processing unit 60, the parameter switching unit 62, the central controller 68, and the automatic mode switching unit 69, are various processors to be described below. The various processors include: a central processing unit (CPU) that is a general-purpose processor functioning as various processing units by executing software (program); a programmable logic device (PLD) that is a processor of which circuit configuration can be changed after manufacture, such as a field programmable gate array (FPGA); a dedicated electrical circuit that is a processor having circuit configuration designed exclusively to perform various types of processing; and the like.

One processing unit may be formed of one of these various processors, or may be formed of a combination of two or more same type or different types of processors (for example, a plurality of FPGAs or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined.

### Explanation of References

10: endoscope system
12: endoscope
12a: insertion part
12b: operation part
12c: bendable part
12d: distal end part
12e: angle knob
13a: mode changeover SW
13b: static-image-acquisition instruction part
13c: zoom operation part
14: light source device
16: processor device
18: display
19: user interface
20: light source unit
20a: violet light emitting diode (V-LED)
20b: blue light emitting diode (B-LED)
20c: green light emitting diode (G-LED)
20d: red light emitting diode (R-LED)
21: light source processor
23: optical path-combination unit
30a: illumination optical system
30b: image pickup optical system
41: light guide
45: illumination lens
46: objective lens
47: zoom lens
47a: zoom drive unit
48: image pickup sensor
50: CDS/AGC circuit circuit
52: A/D converter
53: image acquisition unit
56: digital signal processor (DSP)
58: noise removing unit
60: image processing unit
62: parameter switching unit
63: static image storage unit
66: display controller
68: central controller
69: automatic mode switching unit
70: image-pickup-condition acquisition unit
72: observation portion acquisition section
74: brightness calculation section
76: magnification acquisition section
78: observation distance acquisition section
80: shake amount calculation section
82: mono-light emission switching condition-setting menu

## Claims

1. An endoscope system comprising:
a plurality of semiconductor light sources that emit light having wavelength ranges different from each other;
a light source processor that controls the plurality of semiconductor light sources and performs a control related to a mono-light emission mode where only specific illumination light having a specific light emission ratio is emitted and a control related to a multi-light emission mode where a plurality of types of illumination light, which include first illumination light having a first light emission ratio and second illumination light having a second light emission ratio different from the first light emission ratio, are emitted while being switched according to a specific light emission pattern; and
an image control processor,
wherein in a case where a predetermined mono-light emission switching condition is satisfied and a mode is thus automatically switched to the mono-light emission mode from the multi-light emission mode,
the image control processor automatically switches a mode to the multi-light emission mode from the mono-light emission mode in a case where a preset multi-light emission restart condition is satisfied.

2. The endoscope system according to claim 1,
wherein the image control processor
automatically switches a mode to the multi-light emission mode from the mono-light emission mode in a case where the multi-light emission restart condition is satisfied and a restart allowable condition is satisfied, and
prohibits a mode from being automatically switched to the multi-light emission mode in a case where the multi-light emission restart condition is satisfied and the restart allowable condition is not satisfied.

3. The endoscope system according to claim 2,
wherein the restart allowable condition is a case where a user does not select the mono-light emission mode.

4. The endoscope system according to claim 2 or 3,
wherein the restart allowable condition is a case where a restart allowable time does not elapse yet after a mode is switched to the mono-light emission mode.

5. The endoscope system according to any one of claims 2 to 4,
wherein the restart allowable condition is a case where the multi-light emission mode is implementable.

6. The endoscope system according to any one of claims 2 to 5, further comprising:
a magnification change unit that is used to change a magnification of an object to be observed,
wherein the restart allowable condition is a case where use or non-use of the magnification change unit is not switched.

7. The endoscope system according to any one of claims 2 to 6,
wherein the restart allowable condition is capable of being set by a user.

8. The endoscope system according to any one of claims 1 to 7,
wherein the multi-light emission restart condition is that a change in an image pickup condition about an object to be observed is within an allowable range.

9. The endoscope system according to claim 8,
wherein the multi-light emission restart condition is a condition of a combination of at least one of
a case where the object to be observed returns to a first portion within a certain period of time after being changed to a second portion from the first portion or a case where the object to be observed returns to the second portion within a certain period of time after being changed to the first portion from the second portion,
a case where brightness of the object to be observed is equal to or higher than a first brightness threshold value or is equal to or lower than a second brightness threshold value larger than the first brightness threshold value,
a case where a variation of magnification of the object to be observed is less than a magnification threshold value in a case where the magnification of the object to be observed is changed,
a case where a variation of an observation distance is equal to or smaller than a distance threshold value, or
a case where a shake amount of an observation image obtained from image pickup of the object to be observed is equal to or smaller than a shake-amount threshold value.

10. The endoscope system according to any one of claims 1 to 9,
wherein the image control processor performs a control to display a specific observation image, which is obtained from image pickup of an object to be observed illuminated with the specific illumination light, on a display in a case of the mono-light emission mode and performs a control to display a plurality of observation images including a first observation image, which is obtained from image pickup of the object to be observed illuminated with the first illumination light, and a second observation image, which is obtained from image pickup of the object to be observed illuminated with the second illumination light, on the display while switching the plurality of observation images according to a specific display pattern in a case of the multi-light emission mode.

11. A method of operating an endoscope system including
a plurality of semiconductor light sources that emit light having wavelength ranges different from each other,
a light source processor that controls the plurality of semiconductor light sources and performs a control related to a mono-light emission mode where only specific illumination light having a specific light emission ratio is emitted and a control related to a multi-light emission mode where a plurality of types of illumination light, which include first illumination light having a first light emission ratio and second illumination light having a second light emission ratio different from the first light emission ratio, are emitted while being switched according to a specific light emission pattern,
and an image control processor,
wherein in a case where a predetermined mono-light emission switching condition is satisfied and a mode is thus automatically switched to the mono-light emission mode from the multi-light emission mode,
the image control processor automatically switches a mode to the multi-light emission mode from the mono-light emission mode in a case where a preset multi-light emission restart condition is satisfied.
